# EUROPÄISCHE PATENTSCHRIFT

(11) **EP 1 443 048 B1**
(45) Veröffentlichungstag und Bekanntmachung des Hinweises auf die Patenterteilung: **13.06.2007**
(21) Anmeldenummer: 04001777.4
(22) Anmeldetag: 28.01.2004
(51) Int. Cl.: C07D 487/08

(54) **Entfärbung und Farbstabilisierung von TEDA-Lösung**
Decolorization and color stabilization of TEDA-solutions
Décoloration et stabilsation de la couleur de solutions TEDA

(30) Priorität: 30.01.2003 DE 10303696
(43) Veröffentlichungstag der Anmeldung: 04.08.2004
(73) Patentinhaber: BASF Aktiengesellschaft, 67056 Ludwigshafen (DE)
(72) Erfinder: Ciprian, Jürgen, Dr., 67071 Ludwigshafen (DE); Frauenkron, Matthias, Dr., 67251 Freinsheim (DE); Maurer, Stephan, Dr., 67459 Böhl-Iggelheim (DE); Melder, Johann-Peter, Dr., 67459 Böhl-Iggelheim (DE)
(74) Vertreter: Isenbruck, Günter

(56) Entgegenhaltungen:
- EP-A- 1 070 717
- EP-A- 1 258 485
- DE-A- 2 442 929
- DE-A- 2 611 069

## Beschreibung

Die vorliegende Erfindung betrifft ein Verfahren zur Herstellung von hochreinem Triethylendiamin (TEDA). Dabei wird TEDA verdampft, dieses in dampfförmiger Form in ein flüssiges Lösungsmittel aus der Gruppe der mehrwertigen Alkohole und Ethern davon eingeleitet und die erhaltene Lösung mit einem Adsorbens aus der Gruppe der Aktivkohlen und basischen Anionentauscher in Kontakt gebracht. Die erhaltene TEDA-Lösung ist farbzahlarm und kann als solche in den üblichen Anwendungen ohne weitere Reinigung eingesetzt werden.

TEDA ist ein bedeutender Katalysator für die Herstellung von Polyurethanschäumen. TEDA liegt bei Raumtemperatur als Feststoff vor. Zu seiner Herstellung und auch Reinigung sind verschiedene Verfahren bekannt, u.a. diejenigen, die in den nachfolgend genannten Druckschriften offenbart sind:

DE-A 24 42 929; US 3,297,701; DE-A 36 34 258; DE-A 17 45 627; DE-A 37 18 395; EP-A 111 928; EP-A 382 055; EP-A 842 935, EP-A 842 936; EP-A 831 096; EP-A 952 152 und US 5,741,906.

Die bisher bekannten Verfahren der Herstellung von TEDA führen zu Bildung von Produktgemischen, die neben TEDA noch Wasser, Nebenprodukte wie Piperazin und hochmolekulare Verbindungen sowie das gegebenenfalls bei der Umsetzung eingesetzte Lösungsmittel enthalten. TEDA wird aus diesen Gemischen gewöhnlich durch diskontinuierliche oder kontinuierliche Destillation oder Rektifikation abgetrennt und meist in einem anschließenden Schritt durch Kristallisieren oder Umkristallisieren gereinigt.

TEDA kann ohne Eintreten von Qualitätsverschlechterung, insbesondere der Farbe und Farbstabilität, des Geruchs und der Reinheit, lediglich unter vergleichsweise hohem Aufwand gereinigt bzw. gehandhabt werden.

Für die bekannten, üblichen Anwendungen wird generell ein sehr reines, geruchsloses und reinweißes TEDA gefordert. Die nachfolgend genannten Anmeldungen offenbaren Verfahren, die eine entsprechende TEDA-Qualität liefern sollen:

DE-A 26 11 069; DE-A 28 49 993 und JP-A 49 048 609.

Nachteilig an diesen Verfahren ist, daß sie das TEDA nicht in der gewünschten Qualität liefern.

Die Patentanmeldung EP-A 1 070 717 der Anmelderin betrifft ein Verfahren zur Herstellung von reinem TEDA, bei dem man TEDA verdampft und das dampfförmige TEDA in ein flüssiges Lösungsmittel einleitet sowie das TEDA aus dieser Lösung auskristallisiert.

Die EP-A 1 223 172 der Anmelderin beschreibt ein Verfahren zur Herstellung einer Lösung von reinem TEDA, das dadurch gekennzeichnet ist, daß man TEDA aus einer Mischung enthaltend ein Lösungsmittel oder ein Verdünnungsmittel, das bei Normaldruck einen Siedepunkt im Bereich von 175 bis 250°C aufweist, verdampft und das dampfförmige TEDA in ein flüssiges Lösungsmittel einleitet. Durch anschließendes Auskristallisieren des TEDA aus der so erhaltenen Lösung wird reines TEDA hoher Qualität erhalten.

Mit den Verfahren nach den beiden vorstehend genannten Patentanmeldungen der Anmelderin läßt sich ein TEDA von vorzüglicher Reinheit und Qualität erhalten. Der letzte Verfahrensschritt, also die Kristallisation des TEDA aus der nach dem Quench erhaltenen Lösung, ist in einigen Fällen unumgänglich, beispielsweise dann, wenn TEDA in sehr hoher Qualität und Reinheit gewünscht wird. Soll ein solches TEDA in fester Form vorliegen, ist dieser Kristallisationsschritt oder zumindest ein Isolieren des TEDA aus der Lösung ein obligatorischer Verfahrensschritt und nicht weiter störend. Jedoch wird TEDA häufig in Form einer Lösung in entsprechenden Anwendungen eingesetzt. Dazu muß das zuvor aus einer Lösung auskristallisierte TEDA wieder aufgelöst werden.

Als Lösungsmittel für den TEDA-Quench eignen sich eine Vielzahl organischer Lösungsmittel. Beispiele umfassen aliphatische, cyclische oder acyclische Kohlenwasserstoffe, insbesondere cyclische und acyclische, verzweigte oder unverzweigte Alkane oder Alkangemische, beispielsweise n-Pentan, i-Pentan, Cyclopentan, Hexan, Cyclohexan, Heptan, Octan und Petrolether, chlorierte aliphatische Kohlenwasserstoffe, insbesondere chlorierte Alkane, beispielsweise Dichlormethan, Trichlormethan, aromatische Kohlenwasserstoffe, beispielsweise Benzol, Toluol und Xylole, chlorierte aromatische Kohlenwasserstoffe, beispielsweise Chlorbenzol, Alkohole, beispielsweise Methanol, Ethanol, Ethylenglykol, 1,4-Butandiol und Polyetheralkohole, insbesondere Polyalkylenglykole, beispielsweise Diethylenglykol und Dipropylenglykol, Ketone, insbesondere aliphatische Ketone, beispielsweise Aceton, Methylethylketon und Diethylketon, aliphatische Carbonsäureester, beispielsweise Essigsäuremethylester und Essigsäureethylester, aliphatische Nitrile, beispielsweise Acetonitril und Propionitril, Ether, beispielsweise Dioxan, THF, Diethylether und Ethylenglykoldimethylether sowie Gemische der vorstehend aufgeführten Lösungsmittel.

Als Lösungsmittel für den TEDA-Quench wird vorzugsweise ein aliphatischer Kohlenwasserstoff oder ein Polyalkylenglykol, insbesondere ein gesättigter cyclischer oder acyclischer, aliphatischer Kohlenwasserstoff mit 5 bis 8 C-Atomen, beispielsweise Pentan, Hexan, Cyclohexan, Heptan, Monoethylenglykol, Dipropylenglykol, oder 1,4-Butandiol verwendet. Die optionale Kristallisation des reinen TEDA aus der erfindungsgemäß hergestellten TEDA-Lösung kann nach dem dem Fachmann bekannten Verfahren erfolgen. Die durch eine nachfolgende mehrstufige, vorzugsweise einstufige Kristallisation erhaltenen TEDA-Kristalle sind hochrein.

Bei den Anwendungen, in denen TEDA in Form einer Lösung eingesetzt wird, beispielsweise als Katalysator bei der Polyurethan-Herstellung, wird als bevorzugtes Lösungsmittel ein mehrwertiger Alkohol oder ein Ether eingesetzt. Bevorzugte Lösungsmittel aus dieser Gruppe sind Dipropylenglykol (DPG), 1,4-Butandiol (BDO) und Monoethylenglykol (MEG).

Wenn eines der vorstehend genannten Lösungsmittel eingesetzt wird, kann - bei hohen Anforderungen an die Farblosigkeit und Reinheit - das gasförmige TEDA nicht direkt in dieses gequencht werden, da die dabei entstehende Lösung störende farbzahlgebende Komponenten enthält und zudem in vielen Fällen bei der Lagerung noch weiter nachdunkelt. Aus diesem Grund wird, wie vorstehend beschrieben, das TEDA nach dem Quenchen auskristallisiert und dann erneut in dem gewünschten Alkohol oder Ether aufgelöst. Bei hohen Anforderungen an die Reinheit wird dabei für den Quench vorzugsweise ein niederer aliphatischer Kohlenwasserstoff wie Pentan, Hexan, Cyclohexan oder Heptan verwendet. Nach dem Quench wird mit diesen Lösungsmitteln häufig eine TEDA-Lösung erhalten, die sich bereits aufgrund des eingesetzten Lösungsmittels nicht für die gewünschten Anwendungen eignet, weiterhin sind die erhaltenen Farbzahlen nicht niedrig genug für hohe Anforderungen.

Aus diesem Grund muß das TEDA wieder aus den erhaltenen Lösungen auskristallisiert und danach in dem geeigneten Lösungsmittel aufgelöst werden. Es ist einsichtig, daß bereits aufgrund der vielen dabei durchzuführenden Verfahrensschritte erwünscht ist, über ein einfaches Verfahren zur Herstellung von TEDA-Lösungen für Anwendungen wie die Polyurethan-Herstellung zu verfügen.

Die Aufgabe der vorliegenden Erfindung besteht darin, ein Verfahren zur Herstellung von TEDA-Lösungen in Alkoholen und/oder Ethern zur Verfügung zu stellen, das die vielen Verfahrensschritte nach dem Stand der Technik vermeidet. Vorzugsweise sollen die Lösungsmittel ausgewählt sein aus der Gruppe DPG, BDO und MEG. Insbesondere soll es das Verfahren ermöglichen, durch direkten Quench in das gewünschte Lösungsmittel eine farbzahlarme, den Anforderungen genügende TEDA-Lösung mit hoher Lagerstabilität zu erhalten.

Diese Aufgabe wird gelöst durch ein Verfahren zur Herstellung von TEDA-Lösungen enthaltend ein Lösungsmittel aus der Gruppe der mehrwertigen Alkohole und Ether von mehrwertigen Alkoholen, umfassend
a) Einleiten von gasförmigem TEDA in das Lösungsmittel
b) Behandeln der Lösung mit einem oder mehreren geeigneten Adsorbentien aus der Gruppe der basischen Anionentauscher und Aktivkohlen.

In einer bevorzugten Ausführungsform der vorliegenden Erfindung ist der mehrwertige Alkohol bzw. der Ether ausgewählt aus der Gruppe bestehend aus DPG, BDO und MEG. Weiterhin bevorzugt ist es, wenn die TEDA-Lösung nach dem Kontakt mit den Adsorbentien von diesen abgetrennt wird.

Es wurde gefunden, daß eine TEDA-Lösung in den genannten Lösungsmitteln von hoher Reinheit erhalten wird, wenn nach dem Verdampfen und Einleiten des TEDA in das Lösungsmittel (TEDA-Quench) die erhaltene Lösung mit einem geeigneten Adsorbens aus der Gruppe der basischen Anionentauscher und Aktivkohlen in Kontakt gebracht wird.

Bei den bisher bekannten Verfahren zur Gewinnung von reinem TEDA durch TEDA-Quench wurde das rohe TEDA verdampft, generell destilliert. Das nach dem Quench erhaltene TEDA wies noch Verunreinigungen auf, die eine Kristallisation des TEDA aus der erhaltenen Lösung notwendig machten.

Mit dem erfindungsgemäßen Verfahren dagegen wird nach dem Quench und der Behandlung mit Adsorbentien aus der Gruppe der basischen Anionentauscher und Aktivkohlen eine Lösung erhalten, die ein TEDA hoher Reinheit enthält und die generell direkt eingesetzt werden kann, beispielsweise als Katalysator bei der Polyurethan-Herstellung. Natürlich kann aus der nach dem Quench erhaltenen Lösung das TEDA auch in Substanz nach Kristallisation isoliert werden, wobei jedoch nicht die erfindungsgemäßen Vorteile der wenigen durchzuführenden Verfahrensschritte erreicht werden. Das auf diese Weise zugängliche TEDA weist dann eine hohe Reinheit auf. Es werden derart Reinheitsgrade von > 95 %, vorzugsweise > 99 %, insbesondere > 99,8 %, erreicht.

Das erfindungsgemäße Verfahren wird bis zum Quenchvorgang so durchgeführt, wie es in den Anmeldungen EP-A 1 070 717 und EP-A 1 223 172 der Anmelderin beschrieben wird. Die in den genannten Anmeldungen beschriebenen Verfahrensschritte der Reinigung von TEDA durch Verdampfen und Quenchen sind ein integraler Bestandteil des Verfahrens nach der vorliegenden Anmeldung und durch Bezugnahme in diese eingeschlossen. Die Verfahren werden nachfolgend noch einmal kurz dargestellt.

Durch das Einleiten des dampfförmigen TEDA in ein flüssiges Lösungsmittel (TEDA-Quench) wird die Bildung von unerwünschten Nebenprodukten, die zur Qualitätsminderung führen, entscheidend verringert.

Das Einleiten des dampfförmigen TEDA in das flüssige Lösungsmittel erfolgt in einem Quench-Apparat, vorzugsweise einem Fallfilmkondensator (Dünnschicht-, Rieselfilm- oder Fallstromkondensator) oder in einem Düsenapparat. Dabei kann das dampfförmige TEDA im Gleich- oder im Gegenstrom mit dem flüssigen Lösungsmittel geführt werden. Vorteilhaft ist die Einleitung des dampfförmigen TEDA von oben in den Quenchapparat. Weiterhin vorteilhaft ist die tangentiale Zufuhr des flüssigen Lösungsmittels am Kopf des Fallfilmkondensators oder die Zufuhr des flüssigen Lösungsmittels durch eine oder mehrere Düsen, um eine vollständige Benetzung der Innenwand des Quenchapparates zu erreichen.

Die Menge des verwendeten Lösungsmittels wird nach Zweckmäßigkeitsgesichtspunkten ausgewählt. Im allgemeinen wird so verfahren, daß, je nach Art des Lösungsmittels, Lösungen mit einem TEDA-Gehalt von ca. 1 bis 50 Gew.-%, vorzugsweise 20 bis 40 Gew.-%, erhalten werden.

Im allgemeinen wird die Temperatur im TEDA-Quench durch Temperieren des eingesetzten Lösungsmittels und/oder des Quenchapparates auf 20 bis 100°C, vorzugsweise 30 bis 60°C, eingestellt.

Der Absolutdruck im TEDA-Quench beträgt im allgemeinen 0,5 bis 1,5 bar.

Wird bei der Reinigung des TEDA dieses entsprechend der EP-A 1 223 172 aus einer Mischung mit einem Lösungs- oder Verdünnungsmittel verdampft, weist das Lösungs- oder Verdünnungsmittel vorzugsweise bei Normaldruck einen Siedepunkt von 180 bis 250°C, mehr bevorzugt von 180 bis 230°C, insbesondere von 190 bis 210°C auf.

Als Lösungs- oder Verdünnungsmittel, das die Mischung enthält, aus der das TEDA verdampft wird, eignen sich besonders inerte
- polare aprotische Lösungsmittel beispielsweise Alkyl-2-pyrrolidone, beispielsweise N-Methyl-2-pyrrolidon (NMP), 1-Ethyl-2-pyrrolidon, 1,5-Dimethyl-2-pyrrolidon, 1-Isopropyl-2-pyrrolidon, Ether, beispielsweise Diethylenglykoldiethylether, Triethylenglykoldimethylether und Triethylenglykoldiethylether, Ketone, beispielsweise Acetophenon und Propiophenon, Lactone, beispielsweise γ-Butyrolacton, Sulfoxide, beispielsweise Dimethylsulfoxid, Carbonsäureester, beispielsweise Fumarsäuredimethylester, Nitrile, beispielsweise Benzonitril, und Harnstoffe, beispielsweise 1,3-Dimethyl-imidazolidin-2-on (DMEU) und Tetramethylharnstoff,
- cyclische oder acyclische Kohlenwasserstoffe, insbesondere gesättigte cyclische oder acyclische Kohlenwasserstoffe, beispielsweise Undecan, Dodecan, cis-Dekalin und trans-Decalin,
- chlorierte aliphatische Kohlenwasserstoffe beispielsweise 1-Chloroctan und 1,1-Dichloroctan,
- aromatische Kohlenwasserstoffe, Nitroaromaten und Phenole, beispielsweise Naphthalin, n-Butylbenzol, Phenol, Kresol, Nitrobenzol und Nitrophenol,
- chlorierte aromatische Kohlenwasserstoffe, beispielsweise 1,2-Dichlorbenzol, Benzylchlorid, 1,2,3,4-Tetramethylbenzol und 1,2,3,5-Tetramethylbenzol,
- Alkohole, beispielsweise Benzylalkohol, 2-Ethylhexanol, 1-Octanol, i-Decanol, 1,2-Propandiol, 1,3-Propandiol, Ethylenglykol, Diethylenglykol, 1,2-Propylenglykol, 1,3-Propylenglykol, 1,2-Butandiol, 1,3-Butandiol, 2,3-Butandiol, 1,4-Butandiol, Neopentylglykol, Diethylenglykolmonomethylether und Dipropylenglykol,
- primäre, sekundäre und tertiäre Amine, beispielsweise Tri-n-Butylamin, Benzylamin, Anilin, N-Ethylanilin, N,N-Dimethylanilin und N,N-Diethylanilin,
- N-Alkylamide, beispielsweise N-Methylformamid und N-Methylacetamid
und deren Gemische.

Besonders bevorzugt sind polare aprotische Lösungs- oder Verdünnungsmittel mit einem E^{N}_{T}-Wert von 0,1 bis 0,6, besonders von 0,2 bis 0,5, insbesondere von 0,3 bis 0,45.

(Zur Definition des E^{N}_{T}-Wertes siehe Ch. Reichardt, Solvents and solvent effects in organic chemistry, 2. Auflage, VCH 1988).

Ganz besonders bevorzugte Lösungsmittel sind NMP und Ethylenglykol.

Das Lösungs- oder Verdünnungsmittel kann im einmaligen Durchlauf oder als Kreislauflösung eingesetzt werden.

Die Menge des verwendeten Lösungs- oder Verdünnungsmittel wird nach Zweckmäßigkeitsgesichtspunkten ausgewählt. Im allgemeinen wird so verfahren, dass je nach Art des Lösungs- oder Verdünnungsmittels Lösungen oder Mischungen mit einem TEDA-Gehalt von ca. 1 bis 90 Gew.-%, bevorzugt 40 bis 70 Gew.-%, erhalten werden.

Das Verdampfen des TEDA, optionsweise aus einer Mischung von diesem mit einem Lösungs- oder Verdünnungsmittel kann nach den dem Fachmann geläufigen Verfahren und Bedingungen erfolgen, z.B. in einer Destillations- oder Rektifikationsapparatur, wobei das TEDA gegebenenfalls zusammen mit dem Lösungs- oder Verdünnungsmittel vorgelegt wird.

Das zu reinigende TEDA kann nach bekannten Verfahren, z.B. durch Umsetzung von Monoethanolamin, Diethanolamin, Triethanolamin, Ethylendiamin, Diethylentriamin, Triethylentetramin, Piperazin, N-(2-Hydroxyethyl)-piperazin, N,N'-Bis(2-Hydroxyethyl)-piperazin, N-(2-Aminoethyl)-piperazin, N,N'-Bis(2-Aminoethyl)-piperazin, Morpholin oder Mischungen hiervon an einem Katalysator, beispielsweise Metallpyrophosphate, Metallphosphate, beispielsweise Erdalkalimonohydrogenphosphat, Zeolithe, Zirkoniumphosphate, Al₂O₃, SiO₂, phosphorhaltiges TiO₂ oder ZrO₂ bei erhöhter Temperatur, im allgemeinen 250 bis 450°C, erhalten werden. Üblicherweise beträgt hierbei der Druck 0,1 bis 50, insbesondere 0,1 bis 5 bar. Optional kann die Umsetzung in Gegenwart eines inerten polaren aprotischen Lösungsmittels, wie N-Alkylpyrrolidonen, beispielsweise N-Methylpyrrolidon, Dioxan, THF, Dialkylformamiden, beispielsweise Dimethylformamid, Dialkylacetamiden, beispielsweise Dimethylacetamid und eines inerten Trägergases beispielsweise N₂ oder Ar durchgeführt werden.

Optionsweise kann das TEDA auskristallisiert und durch Fest-Flüssig-Trennung abgetrennt werden.

Im Unterschied zu den genannten Verfahren wird der Quench erfindungsgemäß in einem Lösungsmittel durchgeführt, das ausgewählt ist aus der Gruppe der mehrwertigen Alkohole und Ethern dieser Alkohole. Beispiele für derartige Alkohole umfassen Ethandiol, 1,2-Propandiol, 1,3-Propandiol, 1,2-Butandiol, 1,3-Butandiol, 1,4-Butandiol, Glycerin, Diethylenglykol, Triethylenglykol, Dipropylenglykol, Tripropylenglykol, Trimethylolpropan.

Bei den Ethern der mehrwertigen Alkohole können sämtliche Hydroxylgruppen verethert sein, es ist aber auch möglich, daß nicht sämtliche Hydroxylgruppen verethert sind, beispielsweise können Monoether vorliegen. Die Ether der mehrwertigen Alkohole sind im Allgemeinen von niederen einwertigen Alkoholen abgeleitet, vorzugsweise Methanol oder Ethanol, insbesondere Methanol

Die meist bevorzugten Lösungsmittel für den TEDA-Quench sind ausgewählt aus der Gruppe bestehend aus Monoethylenglykol (MEG), Dipropylenglykol (DPG) und Butandiol (BDO).

Die nach dem Quench erhaltene Lösung enthält in den meisten Fällen farbzahlgebende Komponenten sowie störende Geruchsstoffe und kann bereits aus diesem Grund bei den meisten Anwendungen, insbesondere der Polyurethan-Herstellung, nicht direkt eingesetzt werden. Weiterhin ist auch ein Nachdunkeln der Lösung bei der Lagerung zu beobachten, durch die sich die Qualität der TEDA-Lösung weiter verschlechtert. Durch das Verfahren nach der vorliegenden Erfindung wird es ermöglicht, die farbzahlgebenden wie auch die geruchsbildenden Komponenten zu entfernen und die Lösung so zu stabilisieren, daß ein Nachdunkeln nicht oder nur untergeordnet eintritt.

Dies wird erfindungsgemäß erreicht durch den Kontakt mit einem oder mehreren geeigneten Adsorbentien aus der Gruppe der basischen Anionenaustauscher und Aktivkohlen.

Insbesondere sind die Adsorbentien ausgewählt aus der Gruppe der stark basischen Anionenaustauscher und Aktivkohlen, die für den Einsatz in der Flüssigphase geeignet sind.

Im Rahmen der vorliegenden Erfindung bedeutet "stark basischer Anionenaustauscher" einen Ionenaustauscher, der funktionelle Gruppen enthält, vorzugsweise Cl⁻, OH⁻ oder SO₄⁻.

Werden Aktivkohlen eingesetzt, können diese in Form von Pulverkohlen oder Kornkohlen vorliegen.

Bevorzugte Aktivkohlen sollten eine spezifische Oberfläche von 400 bis 3000 m²/g, insbesondere 700 bis 1500 m²/g, aufweisen.

Beispiele für geeignete Aktivkohlequalitäten sind die unter den nachfolgend genannten Handelsnamen Vertriebenen:
Carbo Tech PAK 1220, Herkunft Carbo Tech,
Chemviron CAL, Herkunft Chemviron,
CPG LF 1240, Herkunft Chemviron,
F300, F400, Herkunft Chemviron.

Beispiele für geeignete basische Anionenaustauscher sind diejenigen, die unter den folgenden Handelsnamen vertrieben werden:
Ambersep 900 OH, (Herkunft Rohm & Haas), MonoPlus M500, MonoPlus MP500, MonoPlus MP62, MonoPlus MP64, MonoPlus M600, MonoPlus M610 (Herkunft jeweils Bayer AG), Amberjet 4200, Amberlite IRA404, (Herkunft jeweils Rohm & Haas).

Es kann eine einzige Aktivkohle bzw. Aktivkohlequalität oder eine Kombination von zwei oder mehreren Aktivkohlen bzw. -qualitäten eingesetzt werden. Ebenso kann ein einziger basischer Anionenaustauscher oder eine Kombination von basischen Anionenaustauschern eingesetzt werden.

In einer bevorzugten Ausführungsform der vorliegenden Erfindung wird eine Kombination aus mindestens einer Aktivkohle und mindestens einem basischen Anionenaustauscher eingesetzt. Es hat sich gezeigt, daß dadurch eine verbesserte Entfärbungsleistung erreicht wird. Die Aktivkohle und der basische Anionenaustauscher können gemeinsam oder in beliebiger Reihenfolge nacheinander zugegeben werden. Vorzugsweise erfolgt eine gemeinsame Zugabe der Adsorbentien, oder die Aktivkohle wird vor dem Anionenaustauscher zugegeben. Es ist insbesondere bevorzugt, die Aktivkohle vor dem Anionenaustauscher zuzugeben.

Ein Vorteil des gemeinsamen Einsatzes von Aktivkohle und basischem Anionenaustauscher liegt darin, daß nicht nur die Farbzahlen der jeweiligen Charge deutlich abgesenkt werden, sondern auch darin, daß die Farbzahlstabilität bei der Lagerung erhöht wird.

Die Temperatur, bei der das erfindungsgemäße Behandeln der TEDA-Lösung mit den Adsorbentien durchgeführt wird, liegt bei Werten von 10 bis 90°C, vorzugsweise 20 bis 80°C, insbesondere 30 bis 70°C.

Das erfindungsgemäße Verfahren kann durchgeführt werden, indem das Adsorbens/die Adsorbentien zu der jeweiligen Charge gegeben und die TEDA-Lösungen und die Adsorbentien innig miteinander in Kontakt gebracht werden, beispielsweise durch Rühren oder Schütteln. Danach wird das Adsorbens vorzugsweise abgetrennt.

In einer bevorzugten Ausführungsform der vorliegenden Erfindung werden die Adsorbentien in Form eines Festbettes angebracht und die zu entfärbende Lösung über das Festbett geleitet. Die Adsorbentien können auch in Form eines Schmelz- oder Wirbelbettes angeordnet sein.

Die Durchführung des Verfahrens kann kontinuierlich, semikontinuierlich oder diskontinuierlich erfolgen. Die bevorzugte kontinuierliche Ausführung ist die in Festbetten mit Karussell-Anordnung, insbesondere mit Regeneration. Die bevorzugte semikontinuierliche Ausführungsform ist die in zwei im Wechsel betriebenen Festbetten. Diskontinuierlich wird das Verfahren vorzugsweise in einem Festbett mit allen Schritten nacheinander betrieben.

In einer bevorzugten Ausführungsform des erfindungsgemäßen Verfahrens wird verbrauchtes Adsorbens regeneriert, wodurch ein erneutes Verwenden bzw. Rückführen in den Prozeß möglich ist. Das Regenerieren sowohl der Aktivkohle als auch des basischen Anionenaustauschers ist mit starken Mineralsäuren und starken Alkalilaugen möglich. Beispiele sind HCl in unterschiedlichen Konzentrationen, beispielsweise 5- und 10%ig, sowie starke Alkalilaugen, beispielsweise NaOH und KOH, in unterschiedlichen Konzentrationen, beispielsweise 5- und 10%ig. Das Regenerieren der Adsorbentien kann kontinuierlich, semikontinuierlich oder diskontinuierlich erfolgen.

Die Erfindung wird nun in den nachfolgenden Beispielen erläutert.

### Beispiel 1

Es wurde eine 33%ige Lösung von TEDA in Dipropylenglykol, die durch Quench erhalten worden war und eine Farbzahl von ca. 300 APHA aufwies, mit den angegebenen Mengen (bezogen auf 100 g Lösung) der jeweiligen Aktivkohle geschüttelt, über 23 Stunden. Die Ergebnisse finden sich in der nachfolgenden Tabelle 1.

| | | Sorbens | | | Farbzahl |
|---|---|---|---|---|---|
| | | Art | Typ | Menge in g je 100g Lösung | APHA |
| **Charge I** | | | | | **357** |
| 1 | Granulat | Aktivkohle | D43/1 | 1.02 | 366 |
| 2 | Granulat | Aktivkohle | D43/1 | 2.95 | 288 |
| 3 | Granulat | Aktivkohle | D43/1 | 4.89 | 247 |
| 4 | Granulat | Aktivkohle | CPG LF 12x40 | 1.00 | 343 |
| 5 | Granulat | Aktivkohle | CPG LF 12x40 | 2.96 | 250 |
| 6 | Granulat | Aktivkohle | CPG LF 12x40 | 4.83 | 203 |
| 7 | Granulat | Aktivkohle | Chemviron CAL | 0.98 | 322 |
| 8 | Granulat | Aktivkohle | Chemviron CAL | 2.92 | 307 |
| 9 | Granulat | Aktivkohle | Chemviron CAL | 4.85 | 191 |
| **Charge II** | | | | | **311** |
| 10 | Pulver | Aktivkohle | PAK 1220 | 5.13 | 69 |
| 11 | Granulat | Aktivkohle | ROW 0,8 supra | 5.07 | 120 |
| 12 | Pulver | Aktivkohle | CAL gemahlen | 5.19 | 70 |
| 13 | Pulver | Aktivkohle | F 300 gemahlen | 5.05 | 92 |
| 14 | Pulver | Aktivkohle | Carbopal PC 250 | 5.10 | 133 |
| 15 | Pulver | Aktivkohle | Carbopal M3 | 5.52 | 99 |
| 16 | Granulat | Molsieb | MS 10 A | 4.68 | 316 |
| 17 | Granulat | Adsorberharz | XAD 4 | 5.28 | 325 |
| **Charge III** | | | | | **402** |
| 18 | Pulver | Aktivkohle | PAK 1220 | 1.02 | 166 |
| 19 | Pulver | Aktivkohle | PAK 1220 | 3.00 | 94 |
| 20 | Pulver | Aktivkohle | PAK 1220 | 5.00 | 66 |
| 21 | Pulver | Aktivkohle | PAK 1000 | 1.08 | 220 |
| 22 | Pulver | Aktivkohle | PAK 1000 | 3.06 | 173 |
| 23 | Pulver | Aktivkohle | PAK 1000 | 5.07 | 151 |
| 24 | Granulat | Zeolith | DAY | 1.05 | 401 |
| 25 | Granulat | Zeolith | DAY | 3.06 | 409 |
| 26 | Granulat | Zeolith | DAY | 5.02 | 414 |
| 27 | stark saures | Ionentauscherharz | Amberlite 252 RFH | 1.01 | 375 |
| 28 | stark saures | Ionentauscherharz | Amberlite 252 RFH | 3.05 | 388 |
| 29 | stark saures | Ionentauscherharz | Amberlite 252 RFH | 5.11 | 421 |
| 30 | stark saures | Ionentauscherharz | Amberlite 252 RFH | 1.02 | 366 |
| 31 | stark saures | Ionentauscherharz | Amberlite 252 RFH | 3.05 | 372 |
| 32 | stark saures | Ionentauscherharz | Amberlite 252 RFH | 5.18 | 343 |
| 33 | stark basisches | Ionentauscherharz | Ambersep 900 OH | 1.00 | 126 |
| 34 | stark basisches | Ionentauscherharz | Ambersep 900 OH | 3.02 | 109 |
| 35 | stark basisches | Ionentauscherharz | Ambersep 900 OH | 4.99 | 98 |
| 36 | schwach basisch | Ionentauscherharz | Lewatit MP 62 WS | 1.05 | 379 |
| 37 | schwach basisch | Ionentauscherharz | Lewatit MP 62 WS | 3.01 | 389 |
| 38 | schwach basisch | Ionentauscherharz | Lewatit MP 62 WS | 5.15 | 392 |
| 39 | Granulat | Silicagel | Sylobead B127 | 1.11 | 379 |
| 40 | Granulat | Silicagel | Sylobead B127 | 3.07 | 385 |
| 41 | Granulat | Silicagel | Sylobead B127 | 5.00 | 391 |
| 42 | Granulat | Silicagel | Sylobead W127 | 1.05 | 385 |
| 43 | Granulat | Silicagel | Sylobead W127 | 3.04 | 396 |
| 44 | Granulat | Silicagel | Sylobead W127 | 5.01 | 399 |

### Beispiel 2

Es wurde die jeweils angegebene Menge 33%iger Quench-Lösung von TEDA in DPG mit der jeweiligen Menge an Adsorbens (Aktivkohle, basische Anionentauscher bzw. Kombination daraus) versetzt und bei 60°C 24 Stunden geschüttelt. Die Ergebnisse zeigt Tabelle 2.

| Versuch | Menge TEDA-Lösung/g | Adsorbens | Menge/g | erhaltene APHA-Farbzahl |
|---|---|---|---|---|
| A | 50,529 | Ambersep 900 OH | 2,551 | 33,8 |
| B | 50,092 | PAK 1200 | 2,598 | 94,5 |
| C | 100,236 | Ambersep 900 OH + PAK 1220 | 2,572/ 2,541 | 32,5 |
| | | | | |
| | | | | |

Es zeigt sich, dass durch eine Kombination von basischem Anionentauscher mit Aktivkohle eine verbesserte Entfärbung gegenüber der Verwendung eines einzelnen Adsorbens beobachtet wird.

### Beispiel 3

Es wurde die jeweils angegebene Menge in 33%iger Quench-Lösung von TEDA in DPG mit einer Farbzahl von 401,7 APHA mit der jeweiligen Menge an Adsorbens (Kombination aus Kornkohle und Anionentauscher) versetzt und bei 60°C 19 Stunden geschüttelt. Die Ergebnisse zeigt Tabelle 3.

**Tabelle 3: Farbzahl von TEDA-Lösungen in DPG nach Behandeln mit Kombination von Kornkohlen mit basischen Anionentauschern**

| Versuch | Menge TEDA-Lösung/g | Adsorbens | Menge/g | erhaltene APHA-Farbzahl |
|---|---|---|---|---|
| A | 100,25 | PAK 1200 +Ambersep 900 OH | 1,010/ 4,020 | 47,3 |
| B | 100,27 | F300 + Ambersep 900 OH | 4,059/ 1,054 | 50,3 |
| C | 100,21 | CAL + Ambersep 900 OH | 1,068/ 4,031 | 37,9 |
| D | 100,12 | CPG LF 12 x 40 + Ambersep 900 OH | 4,066/ 1,099 | 46,3 |
| E | 100,29 | F400 + Ambersep 900 OH | 4,008/ 1,018 | 55,0 |

Das Beispiel zeit, dass man mit dieser Methode Farbzahlen von ≤ 50 APHA erreichen kann.

### Beispiel 4

Die Durchführung erfolgte wie in Beispiel 3 beschrieben, es wurde jedoch eine TEDA/DPG-Lösung einer Farbzahl von 843 APHA verwendet. Die Ergebnisse zeigt Tabelle 4.

**Tabelle 4: Farbzahl von TEDA-Lösungen in DPG nach Behandeln mit Kombination von Kornkohlen mit basischen Anionentauschern**

| Versuch | Menge TEDA-Lösung/g | Adsorbens | Menge/g | erhaltene APHA-Farbzahl |
|---|---|---|---|---|
| A | 100,06 | PAK 1200 +Ambersep 900 OH | 1,003/ 4,032 | 40,4 |
| B | 100,73 | F300 + Ambersep 900 OH | 4,012/ 1,078 | 52,9 |
| C | 100,58 | CAL + Ambersep 900 OH | 2,524/ 2,501 | 36,3 |
| D | 100,83 | CPG LF 12 x 40 + Ambersep 900 OH | 4,033/ 0,993 | 80,9 |
| E | 100,71 | F400 + Ambersep 900 OH | 4,048/ 1,012 | 40,9 |

Das Beispiel zeigt, dass man selbst bei kleinen Farbzahlen von 843 APHA Endfarbzahlen von ≤ 50 APHA erreichen kann.

### Beispiel 5

Es wurden Quench-Lösungen von TEDA in DPG mit verschiedenen Adsorbentien behandelt und die erhaltenen Lösungen anschließend gelagert. Es zeigt sich, daß durch Behandlung mit Aktivkohle und Anionentauscher ein lagerstabiles Produkt entsteht.

**Tabelle 5: Einfluss der Lagerzeit auf die APHA-Farbzahl (Lagerung unter Stickstoff, dunkel, bei Raumtemperatur)**

| Versuch | Adsorbens | Menge Adsorbens/ 100 ml | T/°C | APHA-FZ direkt | APHA-FZ nach Lagerung | APHA-FZ nach Lagerung |
|---|---|---|---|---|---|---|
| A | PAK 1220 | 5 g | 25 | 69 | 93 | 104 |
| | | | | | (1 Monat) | (2 Monate) |
| B* | 1)PAK 1220 | 5 g | 60 | 66 | | |
| | 2) Ambersep | 5 g | 60 | 33 | 31 | |
| | 900 OH | | | | (1,5 Monate) | |
| C* | 1) Ambersep | 5 g | 60 | 98 | 103 | |
| | 900 OH | | 60 | 95 | (1,5 Monate) | |
| | 2) PAK 1220 | 5 g | | | | |
| D | PAK 1220 + | 1g/4g | 60 | 47 | 49 | |
| | Ambersep | | | | (1 Monat) | |
| | 900 OH | | | | | |
| E | CAL + | 1g/4g | 60 | 38 | 40 | |
| | Ambersep | | | | (1 Monat) | |
| | 900 OH | | | | | |
| F | CPG LF | 1g/4g | 60 | 48 | 49 | |
| | 12 x 40 + Ambersep 900 OH | | | | (1 Monat) | |

| | | | | | | |
|---|---|---|---|---|---|---|
| * zuerst Adsorbens 1, dann Adsorbens 2 zugegeben | | | | | | |

## Patentansprüche

1. Verfahren zur Herstellung von TEDA-Lösungen enthaltend ein Lösungsmittel aus der Gruppe der mehrwertigen Alkohole und Ether von mehrwertigen Alkoholen, umfassend
a) Einleiten von gasförmigem TEDA in das Lösungsmittel
b) Behandeln der Lösung mit einem oder mehreren geeigneten Adsorbentien aus der Gruppe der basischen Anionentauscher und Aktivkohlen.

2. Verfahren gemäß Anspruch 1, **dadurch gekennzeichnet, daß** die Adsorbentien ausgewählt sind aus der Gruppe der stark basischen Anionentauscher, Kornkohlen und Pulverkohlen.

3. Verfahren nach Anspruch 1 oder 2, **dadurch gekennzeichnet, daß** der Anionenaustauscher funktionelle Gruppen aufweist, vorzugsweise OH⁻, CL⁻ oder SO₄²⁻.

4. Verfahren nach einem der Ansprüche 1 bis 3, **dadurch gekennzeichnet, dass** die Aktivkohle eine spezifische Oberfläche von 100 bis 3000 m²/g, vorzugsweise 700 bis 1500 m²/g aufweist.

5. Verfahren nach einem der Ansprüche 1 bis 4, **dadurch gekennzeichnet, daß** eine Kombination eines stark basischen Anionentauschers und einer Aktivkohle eingesetzt wird, vorzugsweise diese gemeinsam oder in der Reihenfolge 1) Aktivkohle, 2) Anionentauscher mit der TEDA-Lösung in Kontakt gebracht werden.

6. Verfahren nach einem der Ansprüche 1 bis 5, **dadurch gekennzeichnet, daß** das Lösungsmittel ausgewählt ist aus Ethandiol, 1,2-Propandiol, 1,3-Propandiol, 1,2-Butandiol, 1,3-Butandiol, 1,4-Butandiol, Glycerin, Diethylenglykol, Triethylenglykol, Dipropylenglykol, Tripropylenglykol und Trimethylolpropan und Ethern der genannten Alkohole mit einem niederen einwertigen Alkohol, vorzugsweise Methanol, insbesondere ausgewählt ist aus Ethandiol, 1,2-Butandiol und Diproyplenglykol.

7. Verfahren nach einem der Ansprüche 1 bis 6, **dadurch gekennzeichnet, daß** das gasförmige TEDA durch Verdampfen von rohem TEDA erhalten wird.

8. Verfahren nach einem der Ansprüche 1 bis 7, **dadurch gekennzeichnet, daß** das Adsorbens/die Adsorbentien nach Einsatz regeneriert werden, vorzugsweise mit starken Mineralsäuren oder starken Alkalilaugen.

9. Verfahren nach einem der Ansprüche 1 bis 8, **dadurch gekennzeichnet, daß** das Adsorbens vorliegt in Form eines Festbettes, Schwebebettes oder Wirbelbettes.

10. Verfahren nach einem der Ansprüche 1 bis 9, **dadurch gekennzeichnet, daß** das Verfahren kontinuierlich, diskontinuierlich oder semikontinuierlich durchgeführt wird.

## Claims

1. A process for preparing TEDA solutions comprising a solvent selected from the group consisting of monohydric alcohols and ethers of monohydric alcohols, which comprises
a) passing gaseous TEDA into the solvent,
b) treating the solution with one or more suitable adsorbents from the group consisting of basic anion exchangers and activated carbons.

2. The process according to claim 1, wherein the adsorbents are selected from the group consisting of strongly basic anion exchangers, granulated carbons and pulverulent carbons.

3. The process according to claim 1 or 2, wherein the anion exchanger bears functional groups, preferably OH⁻, Cl⁻ or SO₄²⁻.

4. The process according to any of claims 1 to 3, wherein the activated carbon has a specific surface area of from 100 to 3 000 m²/g, preferably from 700 to 1 500 m²/g.

5. The process according to any of claims 1 to 4, wherein a combination of a strongly basic anion exchanger and an activated carbon is used and these are preferably brought into contact with the TEDA solution either together or in the order 1) activated carbon, 2) anion exchanger.

6. The process according to any of claims 1 to 5, wherein the solvent is selected from among ethanediol, 1,2-propanediol, 1,3-propanediol, 1,2-butanediol, 1,3-butanediol, 1,4-butanediol, glycerol, diethylene glycol, triethylene glycol, dipropylene glycol, tripropylene glycol and trimethylolpropane and ethers of these alcohols with a lower monovalent alcohol, preferably methanol, and is particularly preferably selected from among ethanediol, 1,2-butanediol and dipropyplene glycol.

7. The process according to any of claims 1 to 6, wherein the gaseous TEDA is obtained by vaporization of crude TEDA.

8. The process according to any of claims 1 to 7, wherein the adsorbent/adsorbents are regenerated after use, preferably by means of strong mineral acids or strong caustic alkalis.

9. The process according to any of claims 1 to 8, wherein the adsorbent is present in the form of a fixed bed, a suspended bed or a fluidized bed.

10. The process according to any of claims 1 to 9 which is carried out continuously, batchwise or semicontinuously.

## Revendications

1. Procédé de préparation de solutions de TEDA contenant un solvant du groupe des alcools polyvalents et des éthers d'alcools polyvalents, comprenant :
a) l'introduction de TEDA gazeuse dans le solvant,
b) le traitement de la solution par un ou plusieurs adsorbants appropriés du groupe des échangeurs d'anions basiques et des charbons actifs.

2. Procédé suivant la revendication 1, **caractérisé en ce que** les adsorbants sont choisis dans le groupe des échangeurs d'anions fortement basiques, des charbons en granulés et des charbons en poudre.

3. Procédé suivant la revendication 1 ou 2, **caractérisé en ce que** l'échangeur d'anions présente des groupes fonctionnels, de préférence OH⁻, Cl⁻ ou SO₄²⁻.

4. Procédé suivant l'une quelconque des revendications 1 à 3, **caractérisé en ce que** le charbon actif présente une surface spécifique de 100 à 3000 m²/g, de préférence de 700 à 1500 m²/g.

5. Procédé suivant l'une quelconque des revendications 1 à 4, **caractérisé en ce que** l' on met en oeuvre une combinaison d'un échangeur d'anions fortement basique et d'un charbon actif, ceux-ci étant de préférence mis en contact avec la solution de TEDA ensemble ou dans l'ordre 1) charbon actif, 2) échangeur d'anions.

6. Procédé suivant l'une quelconque des revendications 1 à 5, **caractérisé en ce que** le solvant est choisi parmi l'éthanediol, le 1,2-propanediol, le 1,3-propanediol, le 1,2-butanediol, le 1,3-butanediol, le 1,4-butanediol, le glycérol, le diéthylène glycol, le triéthylène glycol, le dipropylène glycol, le tripropylène glycol et le triméthylolpropane, et des éthers des alcools cités avec un alcool inférieur monovalent, de préférence du méthanol, en particulier le solvant étant choisi parmi l'éthanediol, le 1,2-butanediol et le dipropylène glycol.

7. Procédé suivant l'une quelconque des revendications 1 à 6, **caractérisé en ce que** la TEDA gazeuse est obtenue par évaporation de TEDA brute.

8. Procédé suivant l'une quelconque des revendications 1 à 7, **caractérisé en ce que** le ou les adsorbant(s) est (sont) régénéré(s) après leur mise en oeuvre, de préférence au moyen d'acides minéraux forts ou de lessives alcalines fortes.

9. Procédé suivant l'une quelconque des revendications 1 à 8, **caractérisé en ce que** l'adsorbant se présente sous la forme d'un lit fixe, d'un lit flottant ou d'un lit fluidisé.

10. Procédé suivant l'une quelconque des revendications 1 à 9, **caractérisé en ce que** le procédé est entrepris en mode continu, discontinu ou semi-continu.
